# EUROPEAN PATENT SPECIFICATION

(11) **EP 3 171 857 B1**
(45) Date of publication and mention of the grant of the patent: **19.08.2020**
(21) Application number: 15736304.5
(22) Date of filing: 16.07.2015
(51) Int. Cl.: A61K 9/06, A61K 31/192, A61K 31/327, A61P 17/10

(54) **COMBINATION OF ADAPALENE AND BENZOYL PEROXIDE FOR THE TREATMENT OF SEVERE ACNE**
KOMBINATION AUS ADAPALEN UND BENZOLPEROXID ZUR BEHANDLUNG VON SCHWERER AKNE
COMBINAISON D'ADAPALÈNE ET DE PEROXYDE DE BENZOYLE POUR LE TRAITEMENT DE L'ACNÉ SÉVÈRE

(30) Priority: 25.07.2014 US 201462029043 P
(43) Date of publication of application: 31.05.2017
(73) Proprietor: Galderma Research & Development, 06410 Biot (FR)
(72) Inventor: LEONI, Matthew James, Hampton, New Jersey 08827 (US); GRAEBER, Michael, Lawrenceville, New Jersey 08648 (US); MANNA, Vasant, Ewing, New Jersey 08638 (US)
(74) Representative: Nederlandsch Octrooibureau
(86) International application number: PCT/EP2015/066331
(87) International publication number: WO 2016/012352

(56) References cited:
- WO-A1-03/075908
- WO-A1-03/075908
- WO-A1-2008/006888
- WO-A1-2009/130326
- WO-A1-2015/091828
- FR-A1- 2 909 000
- FR-A1- 2 910 321
- LINDA STEIN GOLD ET AL: "Moderate and Severe Inflammatory Acne Vulgaris Effectively Treated with Single-Agent Therapy by a New Fixed-Dose Combination Adapalene 0.3?%/Benzoyl Peroxide 2.5?% Gel: A Randomized, Double-Blind, Parallel-Group, Controlled Study", AMERICAN JOURNAL OF CLINICAL DERMATOLOGY, vol. 17, no. 3, 5 March 2016 (2016-03-05), pages 293-303, XP055418465, US ISSN: 1175-0561, DOI: 10.1007/s40257-016-0178-4

## Description

### BACKGROUND

### Technical Field:

The present application relates to the combined administration of 0.3% by weight of adapalene and of 2.5% by weight of benzoyl peroxide in a single formula for the treatment of inflammatory acne lesions in a subject afflicted with severe acne.

### Description of Background and/or Related and/or Prior Art:

6-[3-(1-adamantyl)-4-methoxyphenyl]-2-naphthoic acid (referred to hereinbelow as adapalene) is a naphthoic acid derivative with retinoid and anti-inflammatory properties. This molecule was the subject of development for the topical treatment of common acne and of dermatoses sensitive to retinoids.

Adapalene is marketed under the trademark Differin® at a weight concentration of 0.1 %, in the form of an "alcoholic lotion" solution, an aqueous gel and a cream. These compositions are useful for treating acne. WO 03/075908 A1 describes adapalene compositions at a weight concentration of 0.3%, for treating acne.

WO 03/055472 moreover describes stable pharmaceutical compositions comprising adapalene and benzoyl peroxide (BPO) with pH independent gelling agents. Use of such compositions in synergistically treating acne lesions is described in WO 2008/006888 A1. An aqueous gel of 0.1% by weight adapalene and 2.5% by weight benzoyl peroxide is marketed under the trademark Epiduo®.

### SUMMARY OF THE INVENTION

In a first aspect, there is provided herein a regimen for therapeutic treatment of acne lesions in a subject afflicted with severe acne, said regimen comprising topically applying to said subject's skin, as active ingredients, 0.3% by weight of adapalene or a pharmaceutically acceptable salt thereof and 2.5% by weight of benzoyl peroxide, combined in a single formula that delivers said active ingredients together to achieve, in a group of such subjects, a degree of success of at least about 30%, wherein the percents by weight are relative to the weight of the single formula.Said single formula is preferably applied once or twice daily for a period of 8 to 12 weeks.

In a second aspect, there is provided herein a regimen for therapeutic treatment of acne lesions in a subject afflicted with severe acne, said regimen comprising topically applying to said subject's skin, as active ingredients, 0.3% by weight of adapalene or a pharmaceutically acceptable salt thereof and 2.5% by weight of benzoyl peroxide, combined in a single formula that delivers said active ingredients together to reduce the number of inflammatory lesions, wherein the percents by weight are relative to the weight of the single formula. Said single formula is preferably applied once or twice daily for a period of 4 to 12 weeks.

In a third aspect, there is provided herein the combined use of 0.3% by weight of adapalene or a pharmaceutically acceptable salt thereof and 2.5% by weight of benzoyl peroxide, as active ingredients, in the manufacture of a medicament which is a single formula that delivers said active ingredients together, by topical application, in the therapeutic treatment of severe acne, wherein the percents by weight are relative to the weight of the single formula.

In other words, the present invention concerns the use of 0.3% by weight of adapalene or a pharmaceutically acceptable salt thereof and 2.5% by weight of benzoyl peroxide, as active ingredients, for the manufacture of a medicament which is a single formula for topical application that contains both active ingredients for the therapeutic treatment of severe acne, wherein the percents by weight are relative to the weight of said single formula.

In a fourth aspect, there is provided herein a single formula comprising 0.3% by weight adapalene and 2.5% by weight benzoyl peroxide, as active ingredients, said single formula delivering said active ingredients together topically, wherein the percents by weight are relative to the weight of the single formula, and the single formula is for use in the treatment of severe acne, particularly in the treatment of inflammatory lesions in severe acne.

In other words, the present invention concerns a pharmaceutical composition comprising 0.3% by weight of adapalene or a pharmaceutically acceptable salt thereof and 2.5% by weight of benzoyl peroxide, as active ingredients, wherein the percents by weight are relative to the total weight of the composition, for its use by topical administration in the treatment of inflammatory acne lesions. The composition is for use in the treatment of subjects afflicted with severe acne.

It has now surprisingly been demonstrated that a therapeutic combination of 0.3% by weight adapalene and 2.5% by weight benzoyl peroxide (BPO) can produce a degree of success and an improvement in the reduction of inflammatory lesions in patients afflicted with severe acne that is superior to a treatment based on a combination of adapalene 0.1% and BPO 2.5%, while at the same time maintaining the same skin tolerance.

Exemplary embodiments thus feature a formulation of adapalene or a pharmaceutically acceptable salt thereof for the preparation of a pharmaceutical composition, at the set dose of 0.3% by weight, administered together with the set dose of 2.5% by weight benzoyl peroxide (BPO) in a single formula, for the treatment of severe acne, especially to reduce the number of inflammatory acne lesions and to achieve a high degree of success in such treatment.

Acne is initially characterized by keratinization disorders, which are sometimes invisible to the naked eye. Visible acne lesions then develop, while the size of the sebaceous glands and the production of sebum increase.

Exemplary embodiments also concern acne lesions in subjects afflicted with severe acne. The term "acne lesions" means non-inflammatory lesions (open and closed comedones) and inflammatory lesions (papules, pustules, nodules and cysts) caused by acne. According to the present invention, the inflammatory lesions in severe acne patients are treated with a 0.3% adapalene/2.5% benzoyl peroxide single formula as described herein.

More preferably, the pharmaceutical composition is administered by daily or twice daily cutaneous topical application.

The term "adapalene salts" means the salts formed with a pharmaceutically acceptable base, especially mineral bases such as sodium hydroxide, potassium hydroxide and ammonia or organic bases such as lysine, arginine or N-methylglucamine. The term "adapalene salts" also means the salts formed with fatty amines such as dioctylamine and stearylamine.

When a salt of adapalene is used in the single formula (or pharmaceutical composition) of the invention, its amount is determined in order to achieve an amount of adapalene of 0.3% by weight, that is to say, 0.3% by weight of adamantyl)-4-methoxyphenyl]-2-naphthoic acid in acid (non salified) form.

The expression "*combination* of 0.3% by weight adapalene or salt thereof with 2.5% by weight benzoyl peroxide" means a single composition/single formula comprising both adapalene or salt thereof and benzoyl peroxide in the percents indicated, these being percents by weight with respect to the total weight of the single formula/composition.

According to the present invention, the pharmaceutical composition is a fixed combination and comprises, in a pharmaceutically acceptable medium, (i) at least one compound selected from adapalene and pharmaceutically acceptable salts thereof, in an amount equivalent to 0.3% adapalene by weight and (ii) benzoyl peroxide (BPO), in an amount of 2.5% by weight. Preferably, the pharmaceutical composition is intended for a single topical application per day, or twice daily.

The term "pharmaceutically acceptable medium" means a medium that is compatible with the skin, mucous membranes and the integuments of human beings.

The term "fixed combination" should be understood as meaning a combination whose active principles are combined at the specified fixed doses in the same vehicle (single formula) that delivers them together to the point of application. The pharmaceutical composition in the form of a fixed combination is a gel; in this case, the two active principles are dispersed and intimately mixed, during the manufacture, in the same vehicle, which delivers them together during the application of the gel, in the treatment of severe acne.

The treatments have a variable duration, depending on the patient and the severity of his acne. The treatment period may thus run from several weeks to several months. A suitable treatment period or regimen is at least four weeks, preferably from 1 to 6 months and more preferably a duration of about 8 weeks to 3 months (12 weeks) is preferable, the duration of the treatment possibly being prolonged, if necessary, in patients afflicted with severe acne.

### BRIEF DESCRIPTION OF THE DRAWINGS

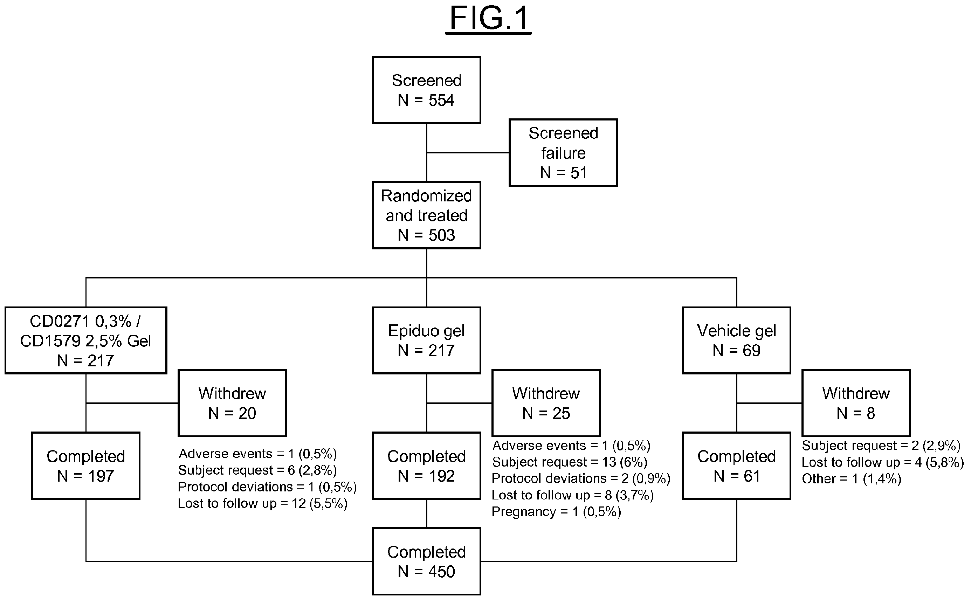

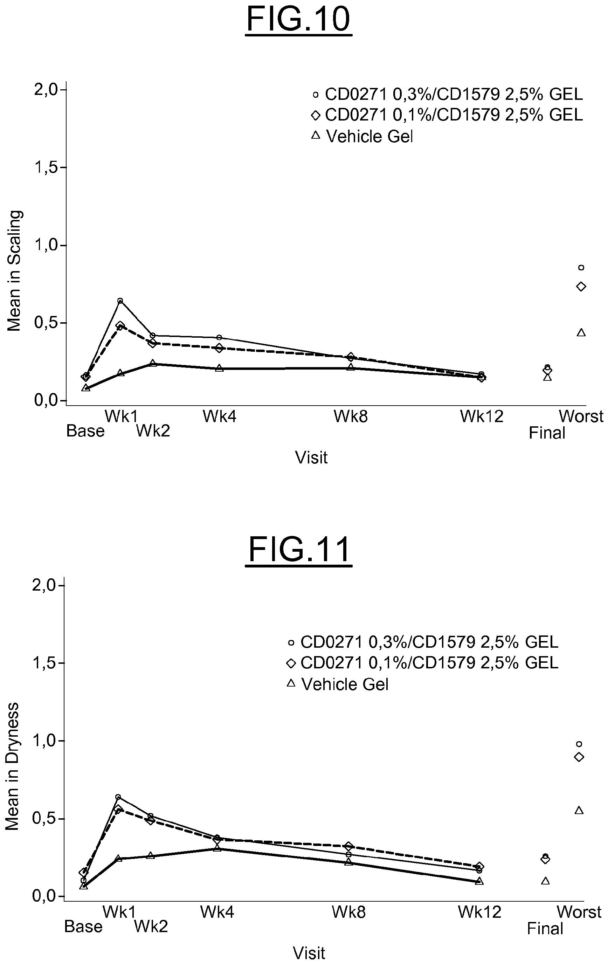

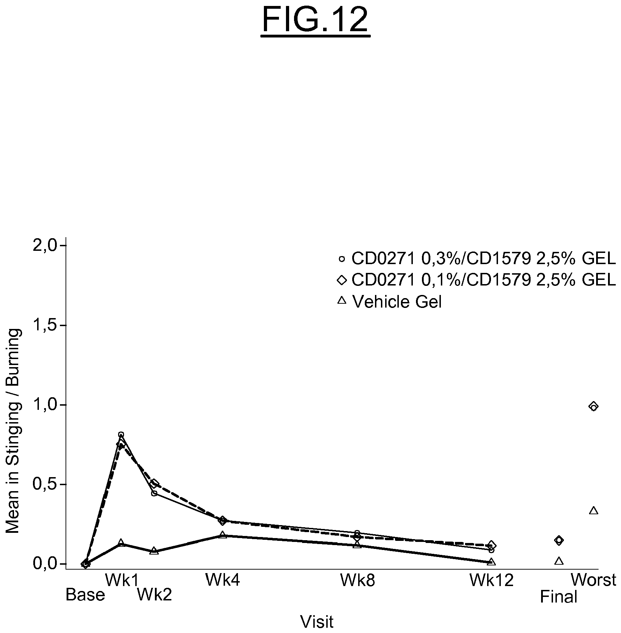

The Figures 1 to 12 annexed to the present disclosure illustrate the results achieved during a clinical study described in the Example hereafter that involves the application of a 0.3% adapalene/2.5% benzoyl peroxide gel, a 0.1% adapalene/2.5% benzoyl peroxide gel marketed under the name EPIDUO®, and the corresponding vehicle gel.
Figure 1 is a schematic of the study disposition.
Figure 2 is a graph of the success rate in percents, over time, comparing 0.3% adapalene/2.5% benzoyl peroxide gel, 0.1% adapalene/2.5% benzoyl peroxide (EPIDUO®) gel and vehicle gel in the Intent-to-Treat (ITT) population (having moderate and severe acne).
Figure 3 is a graph of the mean change in inflammatory lesion count, over time, in the ITT population (having moderate and severe acne), for each of the same three gels.
Figure 4 is a graph of the mean change in non-inflammatory lesion count, over time, in the ITT population (having moderate and severe acne), for each of the same three gels.
Figure 5 is a graph of the success rate and changes in inflammatory and non-inflammatory lesion counts at week 12 in the ITT population (having moderate and severe acne) for the baseline IGA = 4 for each of the same three gels.
Figure 6 is a graph of the success rate (in percents), over time, in subjects with severe acne, of the same three gels.
Figure 7 is a graph of the mean change in inflammatory lesion count, over time, in subjects with severe acne, of the same three gels.
Figure 8 is a graph of the mean change in non-inflammatory lesion count, over time, in subjects with severe acne, for each of the same three gels.
Figure 9 is a graph of the mean incidence of erythema, over time, for each of the same three gels.
Figure 10 is a graph of the mean incidence of scaling, over time, for each of the same three gels.
Figure 11 is a graph of the mean incidence of dryness, over time, for each of the same three gels.
Figure 12 is a graph of the mean incidence of stinging and burning, over time, for each of the same three gels.

### DETAILED DESCRIPTION OF THE INVENTION

In the first and second aspects of the invention disclosed in the SUMMARY hereinabove, the following embodiments are noteworthy, separately or in combination:
(a) the single formula is applied once daily;
(b) the single formula is an aqueous gel;
(c) the aqueous gel comprises a pH independent gelling agent;
(d) the pH independent gelling agent is acrylamide/sodium acryloyldimethyl taurate copolymer and isohexadecane and polysorbate 80 (such as the product sold under the name SIMULGEL 600) or polyacrylamide and isoparaffin and laureth-7 (such as the product sold under the name SEPIGEL 305);
(e) the pH independent gelling agent is an acrylamide/sodium acryloyldimethyl taurate copolymer (such as the product sold under the name SIMULGEL 600);
(f) the aqueous gel further comprises propylene glycol, glycerol and a poloxamer;
(g) the safety profile of said single formula is comparable to that observed under the same conditions for a comparative single formula comprising, as active ingredients, 0.1% by weight adapalene and 2.5% by weight benzoyl peroxide.

In the third and fourth aspects of the invention disclosed in the SUMMARY hereinabove, the following embodiments are noteworthy, separately or in combination:
(a) use in a group of subjects administered said single formula, once or twice daily for a period of 8 to 12 weeks, in particular to achieve a degree of success of at least about 30%;
(b) use in a subject administered said single formula once or twice daily for a period of 4 to 12 weeks, in particular to reduce the number of inflammatory lesions;
(c) use wherein the single formula is an aqueous gel;
(d) use wherein the aqueous gel comprises a pH independent gelling agent;
(e) use wherein the pH independent gelling agent is an acrylamide/sodium acryloyldimethyl taurate copolymer and isohexadecane and polysorbate 80 (such as the product sold under the name SIMULGEL 600) or polyacrylamide and isoparaffin and Laureth-7 (such as the product sold under the name SEPIGEL 305);
(f) use wherein the pH independent gelling agent is an an acrylamide/sodium acryloyldimethyl taurate copolymer (such as the product sold under the name SIMULGEL 600);
(g) use wherein the aqueous gel further comprises propylene glycol, glycerol and a poloxamer;
(h) use wherein the safety profile of said single formula is comparable to that observed, under the same conditions, for a comparative single formula comprising, as active ingredients, 0.1% by weight adapalene and 2.5% by weight benzoyl peroxide.

The pharmaceutical composition is in the form of an aqueous gel.

The term "aqueous gel" means a composition containing, in an aqueous phase, a viscoelastic mass formed from colloidal suspensions (gelling agent).

In a particular embodiment, the aqueous gel contains a "pH independent gelling agent", which means a gelling agent capable of giving the composition a viscosity that is sufficient to keep the adapalene and the benzoyl peroxide in suspension, even under the influence of a variation of pH caused by the release of benzoic acid by the benzoyl peroxide.

Non-limiting examples of pH independent gelling agents that can be mentioned include the gelling agents of the polyacrylamide family, such as a mixture of acrylamide/sodium acryloyldimethyltaurate copolymer and isohexadecane and polysorbate 80 sold under the same SIMULGEL 600 by the company SEPPIC, a mixture of polyacrylamide and isoparaffin C13-12 and laureth-7 such as, for example, the product sold under the name SEPIGEL 305 by the company SEPPIC, the family of acrylic polymers coupled to hydrophobic chains, such as the PEG-150/decyl/SMDI copolymer sold under the name ACULYN 44 (polycondensate comprising at least, as components, a polyethylene glycol containing 150 or 180 mol of ethylene oxide, decyl alcohol and methylenebis (4-cyclohexyl isocyanate) (SMDI), at 35% by weight in a mixture of propylene glycol (39%) and water (26%), the family of modified starches, such as the modified potato starch sold under the name Structure Solanace, or mixtures thereof.

The preferred gelling agents are derived from the polyacrylamide family, such as SIMULGEL 600 or SEPIGEL 305, or mixtures thereof.

The gelling agent as described above can be used in preferential concentrations ranging from 0.1% to 15% and more preferably ranging from 0.5% to 5% by weight with regard to the total weight of the composition.

Alternatively, an aqueous gel can contain alternative or additional gelling agents such as carbomers (carbomer 940 or carbomer 980 or the like), if appropriate.

Exemplary single formula pharmaceutical compositions can also contain inert additives or combinations of these additives, such as
- wetting agents;
- flavor enhancers;
- preservatives such as para-hydroxybenzoic acid esters;
- stabilizers;
- moisture regulators;
- pH regulators;
- osmotic pressure modifiers;
- emulsifiers;
- UV-A and UV-B screening agents; and
- antioxidants, such as α-tocopherol, butylhydroxyanisole or butylhydroxytoluene, superoxide dismutase, ubiquinol or certain metal chelating agents.

Of course, those skilled in the art will take care to select the optional compound(s) to be added to these compositions in such a way that the advantageous properties intrinsically associated with the invention are not, or are not substantially, adversely affected by the envisaged addition.

The gel comprising 2.5% by weight benzoyl peroxide and 0.3% by weight adapalene and gelling agent, especially a pH independent gelling agent, advantageously comprises at least water and can also comprise a pro-penetrating agent and/or a liquid wetting surfactant.

The compositions useful in the present invention can contain one or more pro-penetrating agents in preferential concentrations ranging from 0% to 20% and more preferably ranging from 2% to 6% by weight, relative to the total weight of the composition. They should generally not dissolve the active agents at the percentage used, should not cause any exothermic reactions harmful to the benzoyl peroxide, should aid in the satisfactory dispersion of the active agents, and should have antifoaming properties. Among the pro-penetrating agents preferably used, without this list being limiting, are compounds such as propylene glycol, dipropylene glycol, propylene glycol dipelargonate, lauroglycol and ethoxydiglycol.

A preferred pro-penetrating agent is propylene glycol.

Advantageously, the compositions useful in the present invention can also contain one or more liquid wetting surfactants in preferential concentrations ranging from 0% to 10% and more preferably ranging from 0.1% to 2% by weight, relative to the total weight of the composition. The wetting power is the tendency of a liquid to spread over a surface.

They are preferably surfactants with an HLB (Hydrophilic-Lipophilic Balance) value from 7 to 9, or nonionic surfactants such as polyoxyethylenated and/or polyoxypropylenated copolymers. They should be liquid so as to be readily incorporated into the composition without it being necessary to heat them.

Among the wetting agents that are preferably used, without this list being limiting, are compounds of the Poloxamer family and more particularly Poloxamer 124 and/or Poloxamer 182.

The composition can also comprise any additive usually used in the cosmetics or pharmaceutical field, as noted previously, such as sequestering agents, antioxidants, sunscreens, preserving agents, fillers, electrolytes, humectants, colorants, common mineral or organic acids or bases, fragrances, essential oils, cosmetic active agents, moisturizers, vitamins, essential fatty acids, sphingolipids, self-tanning compounds such as DHA, and calmants and protective agents for the skin such as allantoin. Needless to say, a person skilled in the art will take care to select this or these optional additional compound(s), and/or the amount thereof, such that the advantageous properties of an exemplary composition are not, or are not substantially, adversely affected.

These additives can be present in the composition in a proportion of from 0% to 20% by weight relative to the total weight of the composition.

Examples of sequestering agents that can be mentioned include ethylenediaminetetraacetic acid (EDTA), and also derivatives or salts thereof, dihydroxyethylglycine, citric acid and tartaric acid, or mixtures thereof.

Examples of preserving agents that can be mentioned include benzalkonium chloride, phenoxyethanol, benzyl alcohol, diazolidinylurea and parabens, or mixtures thereof.

Examples of humectants that can be mentioned include glycerol and sorbitol.

Propylene glycol, glycerol and polyoxamer are particularly desirable as additives to the aqueous gels in which the gelling agent is SIMULGEL 600 or SEPIGEL 305 or other pH independent gelling agent.

An exemplary aqueous phase of the aqueous gel can comprise water, a floral water such as cornflower water, or natural mineral or spring water chosen, for example, from eau de Vittel, waters of the Vichy basin, eau d'Uriage, eau de la Roche Posay, eau de la Bourboule, eau d'Enghien-les-Bains, eau de Saint Gervais-les-Bains, eau de Neris-les-Bains, eau d'Allevard-les-Bains, eau de Digne, eau de Maizieres, eau de Neyrac-les-Bains, eau de Lons-le-Saunier, les Eaux Bonnes, eau de Rochefort, eau de Saint Christau, eau des Fumades, eau de Tercis-les-bains, eau d'Avene or eau d'Aix les Bains.

The said aqueous phase can be present in a content of between 10% and 90% by weight and preferably between 20% and 80% by weight, relative to the total weight of the composition.

A particular aqueous gel composition that is preferred herein comprises, in percents by weight relative to the total weight of the aqueous gel:

| | |
|---|---|
| Adapalene | 0.30% |
| Benzoyl peroxide | 2.50% |
| Copolymer of Acrylamide and Sodium Acryloyldimethyl | |
| Taurate & Isohexadecane & Polysorbate 80 (SIMULGEL 600) | 4.00% |
| Sodium docusate | 0.05% |
| Disodium EDTA | 0.10% |
| Glycerol | 4.00% |
| Poloxamer 124 | 0.20% |
| Propylene glycol | 4.00% |
| Purified water | qs 100%. |

Exemplary single formula pharmaceutical compositions are intended for the treatment of severe acne, in particular, common acne (acne vulgaris), comedones, polymorphous acne, nodulocystic acne, acne conglobata, and secondary acne such as solar, drug-related or occupational acne. The expression "severe acne" refers to acne having an [IGA]=4, where IGA means Investigative Global Assessment.

In order to further illustrate exemplary embodiments and the advantages thereof, the following specific Example is given, it being understood that same is intended only as illustrative, not limitative. In the example to follow, the parts and percentages are given by weight with regard to the total weight of each composition, unless otherwise indicated.

### EXAMPLE:

In the clinical study and its summary which follows, the composition variously identified as "CD0271 0.3%/CD1579 2.5% Gel", "CD0271 0.3%/CD1579 2.5% Topical Gel", "adapalene 0.3%/benzoyl peroxide 2.5% topical gel" and "0.3% A/BPO" was an aqueous gel comprising the following, expressed as % by weight/total weight:

| Adapalene | 0.30% |
|---|---|
| Benzoyl peroxide | 2.50% |
| Copolymer of Acrylamide and Sodium Acryloyldimethyl Taurate & Isohexadecane & Polysorbate 80 (SIMULGEL 600) | 4.00% |
| Sodium docusate | 0.05% |
| Disodium EDTA | 0.10% |
| Glycerol | 4.00% |
| Poloxamer 124 | 0.20% |
| Propylene glycol | 4.00% |
| Purified water | qs 100%. |

The composition variously referred to in the clinical study and summary as "Epiduo Gel", "CD0271 0.1%/CD1579 2.5% Gel", "adapalene 0.1%/benzoyl peroxide 2.5% topical gel", and "0.1% A/BPO" was an aqueous gel comprising the following, expressed as % by weight/total weight:

| | |
|---|---|
| Adapalene | 0.10% |
| Benzoyl peroxide | 2.50% |
| Copolymer of Acrylamide and Sodium Acryloyldimethyl Taurate & Isohexadecane & Polysorbate 80 (SIMULGEL 600) | 4.00% |
| Sodium docusate | 0.05% |
| Disodium EDTA | 0.10% |
| Glycerol | 4.00% |
| Poloxamer 124 | 0.20% |
| Propylene glycol | 4.00% |
| Purified water | qs 100%. |

The composition referred to in the clinical study and summary as "Vehicle Gel" or "vehicle" had the same composition as the two test gels described above, but contained no adapalene and benzoyl peroxide.

The study disclosed hereafter shows that an adapalene 0.3% / benzoyl peroxide 2.5% topical gel allows achieving superior results in the treatment of moderate and severe acne vulgaris. This study compared the efficacy and safety of adapalene 0.3%/benzoyl peroxide 2.5% (0.3% A/BPO) topical gel versus vehicle in subjects with moderate and severe acne (overall population [OP]), and in a subpopulation of the OP (severe acne subjects only) (severe population [SP]). The study also compared 0.3% A/BPO versus adapalene 0.1%/benzoyl peroxide 2.5% (0.1% A/BPO) topical gel in the SP.

This multicenter, randomized, double-blind, parallel-group, vehicle- and active-controlled study included a total of 503 subjects. Subjects were randomized to apply 0.3% A/BPO (n=217), 0.1% A/BPO (n=217) or vehicle (n=69) once daily for 12 weeks. The OP included subjects with moderate (investigator global assessment [IGA]=3) and severe (IGA=4) acne, while the SP consisted only of subjects with severe disease (IGA=4). Co-primary efficacy endpoints included success rate (at least 2-grade improvement on IGA) and change in inflammatory (IN) and non-inflammatory (NIN) lesion count from baseline to week 12.

In the OP, 0.3% A/BPO was superior to vehicle in success rate (33.7% vs. 11.0%), and changes in IN (-27.0 vs. -14.4) and NIN lesion counts (-40.1 vs. -18.4), as well as percent changes in IN (-68.7% vs. - 39.2%) and NIN lesion counts (-68.3% vs. -37.3%, respectively; all p<.001). Similarly, in the SP, 0.3% A/BPO was superior to vehicle in success rate (31.9% vs. 11.8%; p=.029), and changes in IN (-35.1 vs. -15.4) and NIN lesion counts (-45.6 vs. -17.2), as well as percent changes in IN (-74.4% vs. -33.0%) and NIN lesion counts (-72.0% vs. -30.7%, respectively; all p<.001). Moreover, 0.3% A/BPO showed a positive trend toward superiority over 0.1% A/BPO in the SP with a 11.4% difference (95% confidence intervals [CI]: -0.5%, 23.2%) in success rate, and -3.25 [CI: -7.15, 0.64] and -2.51 [CI: -8.08, 3.06] for changes in IN and NIN lesion counts.

In terms of safety, 0.3% A/BPO was well tolerated, with a similar local tolerability profile to that of 0.1% A/BPO. Treatment-related adverse events (AEs) (15 AEs in 12 subjects [5.5%] vs. 2 AEs in 1 subject [0.5%], respectively) were mild to moderate in severity. Only 1 subject (0.2%) in the 0.3% A/BPO group discontinued treatment due to an AE (atopic dermatitis flare) and no serious AEs were reported.

Adapalene 0.3%/BPO 2.5% topical gel showed superior efficacy to vehicle in the general population, with greater efficacy shown in subjects with severe acne. The safety profile of 0.3% A/BPO was acceptable and comparable to that observed for 0.1 % A/BPO.

### EXECUTIVE SUMMARY OF THE STUDY:

This study was a multicenter, randomized, double-blind, parallel-group, vehicle and active-controlled, 12 week study investigating the efficacy and safety of CD0271 0.3%/CD1579 2.5% topical gel applied once daily versus Epiduo gel (adapalene/BPO) and Vehicle Gel applied topically once daily in subjects with moderate and severe acne vulgaris. This study has been reviewed and endorsed by FDA as part of a Special Protocol Assessment.

A total of 503 subjects from 31 clinical sites were randomized to the study with 217, 217 and 69 subjects randomized into the CD0271 0.3%/CD1579 2.5% topical gel, Epiduo Gel and Vehicle arms, respectively. All 503 subjects were included in the ITT and Safety population, and 459 subjects were included in the PP population. The study was stratified with approximately 50% of the subjects rated IGA=3-moderate (251 subjects) and 50% rated IGA=4-severe (252 subjects).

Three efficacy objectives were pre-specified in a hierarchical order. The 3 objectives were to be tested in the order listed below, each conditionally depending on the success of the preceding objective.

1. To demonstrate the superiority in efficacy of CD0271 0.3%/CD1579 2.5% Gel versus Gel Vehicle in the treatment of acne vulgaris for up to 12 weeks in the overall population of moderate (IGA=3) and severe acne (IGA=4).

2. To demonstrate the superiority in efficacy of CD0271 0.3%/CD1579 2.5% Gel versus Gel Vehicle in the subgroup of subjects with severe acne (IGA=4).

3. To assess the superiority of CD0271 0.3%/CD1579 2.5% versus Epiduo Gel in the subgroup of subjects with severe acne (IGA=4) by the point estimate of the numerical difference versus Epiduo Gel and the 95% Cl of the difference.

The study was considered as positive in efficacy outcome, if objectives (1) and (2) were met.

In addition, tolerability and safety were assessed during the study.

### Summary of results:

### Results of the primary objectives were as follow:

Superiority of CD0271 0.3%/CD1579 2.5% Gel over vehicle gel was demonstrated in the overall study population (IGA 3 moderate and IGA 4 severe) for Success Rate (subjects rated 'Clear' or 'Almost Clear' with at least 2-grade improvement in IGA) (33.7% vs. 11.0%, p <0.001) and for Changes in Inflammatory (-27.04 vs. -14.40, p <0.001) and Non-Inflammatory Lesion Counts (-40.18 vs. -18.47, p <0.001).

In the severe stratum (IGA=4), Statistical superiority of CD0271 0.3%/CD1579 2.5% Gel over vehicle gel was demonstrated for Success Rate (subjects rated 'Clear' or 'Almost Clear' with at least 2-grade improvement on IGA) (31.9% versus 11.8%, p=0.029) and for Changes in Inflammatory (-35.17 vs. -15.46, p <0.001) and Non-Inflammatory Lesion Counts (-45.61 vs. -17.25, p <0.001).

The differences and 95% Confidence Intervals (CIs) of CD0271 0.3%/CD1579 2.5% Gel from Epiduo Gel in the severe stratum (IGA=4), were estimated to be 11.4% [-0.5%, 23.2%] for success rate, -3.25 [-7.15, 0.64] for changes in Inflammatory lesion counts; and -2.51 [-8.08, 3.06] for changes in Non-Inflammatory lesion counts, with all endpoints showing a positive trend toward superiority over Epiduo Gel. The study was not powered to demonstrate statistical significance for this comparison.

### Secondary efficacy objectives:

Statistical superiority of CD0271 0.3%/CD1579 2.5% Gel over vehicle gel in the overall population was demonstrated for percent Changes in Inflammatory (-68.70% vs. -39.23%, p-value <0.001) and Non-Inflammatory Lesion Counts (-68.34% vs. -37.38%, p-value <0.001).

### Safety objectives:

CD0271 0.3%/CD1579 2.5% was well tolerated in this study. The local tolerability profile was similar to that of Epiduo Gel, showing a peak of signs and symptoms at Week 1, followed by progressive resolution with continued treatment. The few Treatment Emergent Adverse Events (TEAEs) were generally dermatological in nature and mild to moderate in severity. Only one subject (0.2%) in the CD0271 0.3%/CD1579 2.5% Gel group was discontinued due to AE (atopic dermatitis flare). No serious adverse events were reported in the CD0271 0.3%/CD1579 2.5% group.

### General conclusion:

This study demonstrated that CD0271 0.3%/CD1579 2.5% Gel was superior to vehicle in the co-primary endpoints of IGA success rate (clear/almost clear and at least two grade change) and change in Inflammatory and Non-inflammatory lesions counts. The statistical significance was consistently high (p<0.001) and the outcome clinically compelling. The study is robust, presenting consistent results in the PP population and sensitivity analyses.

In the subgroup of subjects with severe acne (IGA=4), CD0271 0.3%/CD1579 2.5% Gel was superior to Vehicle in IGA success rate (p=0.029), change in Inflammatory (p<0.001) and Non-Inflammatory lesion counts (p<0.001). The outcome was supported by robust sensitivity analyses.

A trend of numerical superiority of CD0271 0.3%/CD1579 2.5% Gel compared to Epiduo Gel in IGA success rate, change in Inflammatory and Non-Inflammatory lesion counts was observed in subjects with severe acne (IGA=4).

CD0271 0.3%/CD1579 2.5% Gel was well tolerated with an acceptable safety profile.

### STUDY DESIGN:

This was a multicenter, randomized, double-blind, parallel-group, vehicle and active controlled, 12 week study investigating the efficacy and safety of CD0271 0.3%/CD1579 2.5% topical gel applied once daily versus Epiduo gel and Vehicle Gel applied once daily in subjects with moderate and severe acne vulgaris.

Randomization was stratified by investigational sites and IGA severity such that 50 % of the subjects were to have IGA scores of 3 and 4, respectively. The inclusion of the subjects with severe acne was intended to allow for the demonstration of efficacy in that subgroup of subjects.

Qualified subjects were randomized in a 3:3:1 ratio to receive either CD0271 0.3%/CD1579 2.5% topical gel, Epiduo Gel or Vehicle Gel for 12 weeks.

Major entry criteria included: clinical diagnosis of moderate to severe facial acne with a score of 3 (moderate) or 4 (severe) on the IGA scale and presence of 20 to 100 Inflammatory lesions, 30 to 150 Non-Inflammatory lesions on the face (including the nose), and up to 2 nodules on the face. Subjects presenting with both facial and truncal acne vulgaris were to participate in the study; however, only facial acne lesions were evaluated

### SUMMARY OF STUDY RESULTS:

### Study Characteristics:

A total of 503 subjects from 31 clinical sites were randomized to CD0271 0.3%/CD1579 2.5% Topical Gel, Epiduo gel or Vehicle Gel: 217, 217 and 69 subjects in the CD0271 0.3%/CD1579 2.5% topical gel, Epiduo Gel or Vehicle Gel arms, respectively.

All 503 subjects were included in the ITT and Safety population, and 459 subjects were included in the PP population. Study disposition is shown in Figure 1.

A total of 53 subjects (10.5%) discontinued the study prematurely. Two discontinuations were due to Adverse Events (Atopic Dermatitis Flare in the CD0271 0.3%/CD1579 2.5% topical gel group and Worsening of Acne in the Epiduo Gel group).

Treatment groups were comparable with respect to the demographic and baseline characteristics. Of the total 503 subjects, 263 (52.3%) were female, 389 (77.3%) were Caucasians, and the mean age was 19.6 years (median 17.0 years).

### Efficacy:

### Primary Efficacy analysis:

The Co-primary efficacy endpoints were defined as:
1. Success Rate, the percentage of subjects with an IGA of clear or almost clear (and therefore at least a 2-grade improvement from Baseline at Week 12 Intent-to-treat [ITT]);
2. Change in Inflammatory Lesion Count from Baseline to Week 12 (ITT);
3. Change in Non-inflammatory Lesion Count from Baseline to Week 12 (ITT);

Statistical analyses were performed to compare and interpret in a stepwise manner:
CD0271 0.3%/CD1579 2.5% Gel versus Topical Gel Vehicle in the overall population of moderate and severe acne by formal inferential hypothesis testing.
CD0271 0.3%/CD1579 2.5% Gel versus Topical Gel Vehicle in the subgroup of subjects with severe acne (Investigator's Global Assessment IGA=4) by formal inferential hypothesis testing.
CD0271 0.3%/CD1579 2.5% versus Epiduo Gel (adapalene/BPO) in the subgroup of subjects with severe acne (IGA=4) by using the point estimate of the numerical difference versus Vehicle Gel and the 95% CI of the difference.

The Multiple Imputation (MI) procedure was used as the primary imputation method to impute for missing efficacy data.

The primary efficacy analyses (Week 12 MI, ITT) showed (Table 2) CD0271 0.3%/CD1579 2.5% Gel was statistically more effective than Vehicle Gel in the co-primary endpoints:
1. Success Rate (MI, ITT) (difference between CD0271 0.3%/CD1579 2.5% Gel vs. Vehicle is 22.7%, with 95% CI of [12.8%, 32.6%], p<0.001)
2. Change in Inflammatory Lesion Count from Baseline to Week 12 (MI, ITT) (Least Square Mean difference between CD0271 0.3%/CD1579 2.5% Gel vs. Vehicle are -12.64 lesions with 95% CI of [-15.82, -9.46], p<0.001),
3. Change in Non Inflammatory Lesion Count from Baseline to Week 12 (MI, ITT) (Least Square Mean difference between CD0271 0.3%/CD1579 2.5% Gel vs. Vehicle are -21.71 lesions with 95% CI of [-26.66, -16.76], p< 0.001).

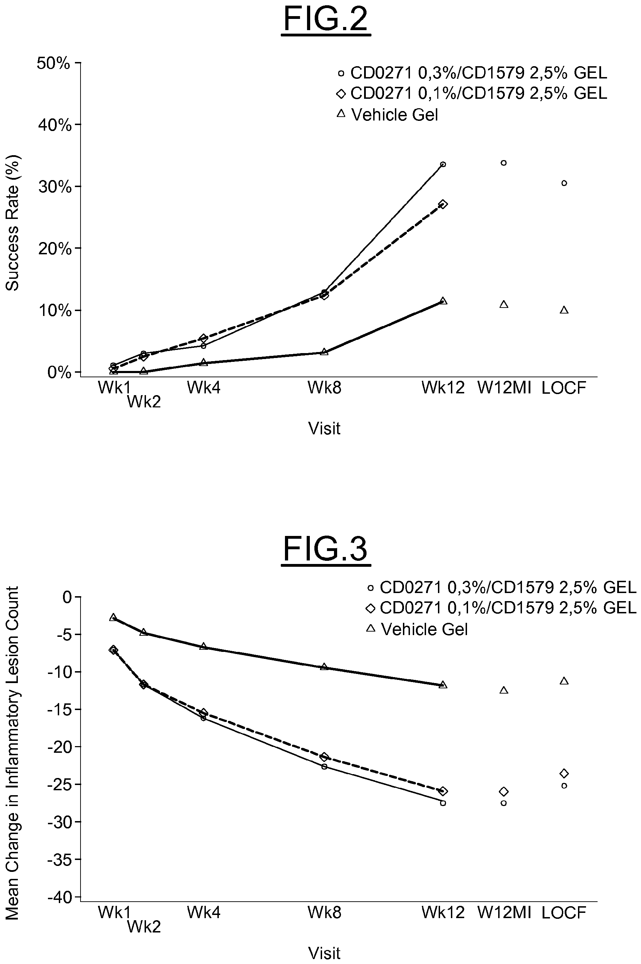

The primary efficacy analyses results were also confirmed in the PP analyses and sensitivity analyses using traditional LOCF imputation method for missing data. Results are shown in the Table 1 below and Figures 2 to 4.

**Table 1- Summary of the primary analysis in the full population (MI, ITT)**

| Efficacy Parameters | CD0271 0.3%/CD 1579 2.5% Gel | Epiduo Gel | Vehicle Gel | CD0271 0.3%/CD 1579 2.5% Gel vs. Vehicle Gel | |
|---|---|---|---|---|---|
| | | | | Treatment Difference (95% CI) | p-values |
| Full Population (Baseline IGA = 3 and 4) | | | | | |
| # of Subjects | N=217 | N=217 | N=69 | - | - |
| Success Rate | 33.7% | 27.3% | 11.0% | 22.7% (12.8%, 32.6%) | <0.001 |
| Change in Inflammatory Lesion (LS Mean change (SE) | -27.04 (0.846) | -26.72 (0.822) | -14.40 (1.460) | -12.64(-15.82, - 9.46) | <0.001 |
| Change in Non-inflammatory Lesions (LS Mean change (SE) | -40.18 (1.332) | -39.00 (1.277) | -18.47(2.270) | -21.71(-22.66, - 16.76) | <0.001 |

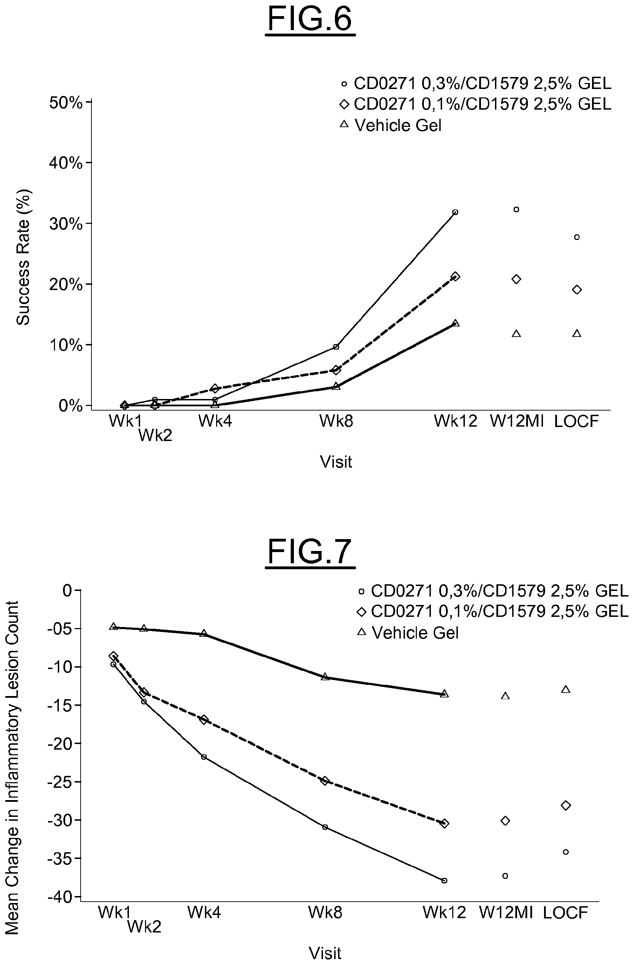

In the severe stratum (IGA=4), the primary efficacy analyses (Week 12 MI, ITT) also showed (Table 2 and Figures 6 to 8) that CD0271 0.3%/CD1579 2.5% Gel was statistically more effective than Vehicle Gel for Success Rate (31.9% vs. 11.8%; p=0.029) and Change in Inflammatory (-35.2 vs. -15.5; p<0.001) and Non Inflammatory Lesion Counts (-45.6 vs. -17.3; p<0.001) at Week 12.

The primary analyses results for subjects with baseline IGA=4 (severe) were confirmed by consistent results in the sensitivity analyses.

A trend of numerical superiority of CD0271 0.3%/CD1579 2.5% Gel compared to Epiduo Gel in IGA success rate (11.4% difference), change in Inflammatory lesion count (3.25 difference) and change in Non-Inflammatory lesion count (2.51 difference) was observed in subjects with severe acne (IGA=4).

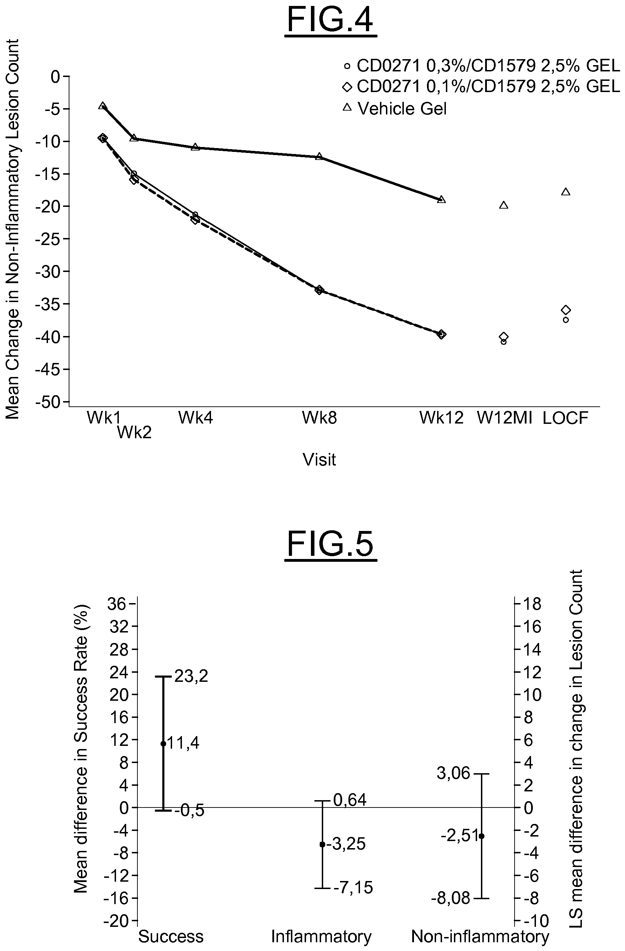

No formal hypothesis testing was planned for such comparison, as the sample size was deemed to be too small for this comparison between the two active treatments. However, the treatment difference estimates and their 95% confidence intervals show a positive trend, close to significance. Confidence intervals are illustrated in the Figure 5.

**Table 2- Summary of the primary analysis in the severe stratum (Baseline IGA = 4 (MI, ITT)**

| Efficacy Parameters | CD0271 0.3%/CD 1579 2.5% Gel | Epiduo Gel | Vehicle Gel | CD0271 0.3%/CD 1579 2.5% Gel vs. Vehicle Gel | | CD0271 0.3%/CD 1579 2.5% Gel vs. Epiduo gel |
|---|---|---|---|---|---|---|
| | | | | Treatment Effect Difference (95%CI) | p-values | Treatment Effect Difference (95% CI) |
| Subject with Severe Acne (Baseline IGA = 4) | | | | | | |
| # of Subjects | N=106 | N=112 | N=34 | | | |
| Success Rate | 31.9% | 20.5% | 11.8% | 20.1% (6.0%, 34.2%) | 0.029 | 11.4% (-0.5%, 23.2%) |
| Change in Inflammatory Lesion (LS Mean change (SE) | -35.17 (1.407) | -31.92 (1.320) | -15.46 (2.297) | -19.71 (-24.81, -14.62) | <0.001 | -3.25 (-7.15, 0.64) |
| Change in Non-inflammatory Lesions (LS Mean change (SE) | -45.61 (2.058) | -43.10 (1.847) | -17.25 (3.337) | -28.36 (-35.64, -21.08) | <0.001 | -2.51 (-8.08,3.06) |

### Percent Changes in Inflammatory and Non-Inflammatory Lesion Counts (Secondary analyses)

Control of multiplicity linked to the two secondary endpoints was achieved by using the Hochberg procedure. The analyses of these secondary efficacy endpoints were also statistically significant versus vehicle in both the combined overall population (Table 3) and in the severe population (Table 4) (p<0.001).

**Table 3- Summary of Percent Change in Inflammatory lesion count and Non-Inflammatory in the full population (MI, ITT)**

| Secondary Analyses(MI, ITT) | CD0271 0.3%/CD 1579 2.5% Gel | Epiduo Gel | Vehicle Gel | CD0271 0.3%/CD 1579 2.5% Gel vs. Vehicle Gel Unadjusted p-values |
|---|---|---|---|---|
| Full Population (Baseline IGA = 3,4) | | | | |
| # of Subjects | N=217 | N=217 | N=69 | |
| Mean Percent Change in Inflammatory Lesions | -68.70 | -69.31 | -39.23 | <0.001 |
| Mean Percent Change in Non-inflammatory Lesions | -68.34 | -68.00 | -37.38 | <0.001 |

**Table 4- Summary of Percent Change in Inflammatory and Non-Inflammatory Lesion Counts in the subjects with severe acne (Baseline IGA = 4) (MI, ITT)**

| Secondary Analyses(MI, ITT) | CD0271 0.3%/CD 1579 2.5% Gel | Epiduo Gel | Vehicle Gel | CD0271 0.3%/CD 1579 2.5% Gel vs. Vehicle Gel Unadjusted p-values |
|---|---|---|---|---|
| Subject with Severe Acne (Baseline IGA = 4) | | | | |
| # of Subjects | N=106 | N=112 | N=34 | |
| Mean Percent Change in Inflammatory Lesions | -74.44 | -68.01 | -33.00 | <0.001 |
| Mean Percent Change in Non-inflammatory Lesions | -72.05 | -68.41 | -30.79 | <0.001 |

### Safety:

### Treatment Duration:

The planned treatment period duration as per protocol was 12 weeks. In this study the actual mean treatment duration was 79.0 days for CD0271 0.3%/CD1579 2.5% Gel, 78.3 days for Epiduo Gel, and 78.4 days for Vehicle Gel.

### Adverse Events:

This section on AEs summarizes the overall AEs, followed by Related AEs, AEs leading to discontinuation, severe AEs and AEs of special interest.

### Overall Adverse Events:

A total of 167 AEs were reported in 105 subjects during the study: 50 subjects (23.0 %) in the CD0271 0.3%/CD1579 2.5% Gel. arm, 42 subjects (19.4%) in the Epiduo arm and 13 subjects (18.8%) in the Vehicle arm (Table 5).

**Table 5- Summary of Overall Adverse Events (Safety Population)**

| | **CD0271 0.3%/CD 1579 2.5% Gel (N=217)** | **Epiduo Gel (N=217)** | **Vehicle Gel (N=69)** | **Total (N=503)** |
|---|---|---|---|---|
| Total Number of AE(s) | 82 | 66 | 19 | 167 |
| Subjects with AE(s) | 50 (23.0%) | 42 (19.4%) | 13 (18.8%) | 105 (20.9%) |
| Subjects with Related AE(s) | 12 (5.5%) | 1 (0.5%) | 0 | 13 (2.6%) |
| Subjects with severe AE(s) | 0 | 0 | 1 (1.4%) | 1 (0.2%) |
| Subjects with SAE(s) | 0 | 1 (0.5%) | 0 | 1 (0.2%) |
| Subjects with AE(s) Leading to Discontinuation | 1 (0.5%) | 1 (0.5%) | 0 | 2 (0.4%) |
| Subjects with AE(s) of Special Interest | 1 (0.5%) | 0 | 0 | 1 (0.2%) |

In the CD0271 0.3%/CD1579 2.5% Gel group, the most common AEs (Table 6a) (≥ 2% in any group) were Nasopharyngitis [14 (6.5%) subjects], Skin irritation [9 (4.1%) subjects].

In the Epiduo gel group, the most common AEs (≥ 2%) (Table 6a) were Nasopharyngitis [11 (5.1%) subjects], Upper respiratory tract infection [5 (2.3%) subjects].

In the Vehicle Gel group, Upper respiratory tract infection was the only AE reported in more than 2% of subjects [4 (5.8%) subjects] (Table 6a).

Adverse events (AEs) in the Skin and Subcutaneous Tissue Disorders category were observed more frequently in subjects in the CD0271 0.3%/CD1579 2.5% Gel group [20 subjects (9.2%)] compared to the Epiduo Gel group [8 subjects (3.7%)] and the Vehicle Gel group [2 subjects (2.9%)].

**Table 6a - Summary of Most Common Adverse Events (≥ 2%) (All Safety Population)**

| | **CD0271 0.3%/CD 1579 2.5% Gel (N=217)** | **Epiduo Gel (N=217)** | **Vehicle Gel (N=69)** | **Total (N=503)** |
|---|---|---|---|---|
| **Infections and infestations** | **23 (10.6%)** | **24 (11.1%)** | **8 (11.6%)** | **55 (10.9%)** |
| Nasopharyngitis | 14 (6.5%) | 11 (5.1%) | 1 (1.4%) | 26 (5.2%) |
| Upper respiratory tract infection | 1 (0.5%) | 5 (2.3%) | 4 (5.8%) | 10(2.0%) |
| | | | | |
| **Skin and subcutaneous tissue disorders** | **20 (9.2%)** | **8 (3.7%)** | **2 (2.9%)** | **30 (6.0%)** |
| Skin irritation | 9 (4.1%) | 1 (0.5%) | 0 | 10 (2.0%) |

Safety profiles are similar in the subgroup of subjects with severe acne (baseline IGA= 4), as compared to the overall population in the three treatment groups.

In subgroup of subjects with severe acne (baseline IGA=4), the most common adverse events (≥ 2% in any group) (Table 6b) in the Skin and Subcutaneous Tissue Disorders category were observed similarly frequently in subjects in the CD0271 0.3%/CD1579 2.5% Gel group [6 subjects (5.7%)] compared to the Epiduo Gel group [6 subjects (5.4%)], both are more frequently than Vehicle Gel group [1 subject (2.9%)]. Interestingly, very few subjects in the severe stratum reported skin irritation with the CD0271 0.3%/CD1579 2.5% Gel group.

**Table 6b - Summary of Most Common Adverse Events (≥ 2%) (Safety Population, IGA=4)**

| | **CD0271 0.3%/CD 1579 2.5% Gel (N=106)** | **Epiduo Gel (N=112)** | **Vehicle Gel (N=34)** | **Total (N=252)** |
|---|---|---|---|---|
| **Infections and infestations** | **12 (11.3%)** | **11 (9.8%)** | **4 (11.8%)** | **27 (10.7%)** |
| Nasopharyngitis | 8 (7.5%) | 5 (4.5%) | 1 (2.9%) | 14 (5.6%) |
| Influenza | 2 (1.9%) | 1 (0.9%) | 1(2.9%) | 4 (1.6%) |
| | | | | |
| **Skin and subcutaneous tissue disorders** | **6 (5.7%)** | **6 (5.4%)** | **1 (2.9%)** | **13 (5.2%)** |
| _{S}kin irritation | 2 (1.9%) | 1 (0.9%) | 0 | 3 (1.2%) |

### Related Adverse Events:

A total of 17 AEs considered related to study medication were reported in 13 subjects (2.6%) during the study: 15 related AEs were reported in 12 subjects (5.5%) in the CD0271 0.3%/CD1579 2.5% Gel arm, and 2 related AEs were reported in 1 subject (0.5%) in the Epiduo arm and no related AEs were reported in the vehicle arm (Table 7).

Of the 17 AEs considered related to study medication, 10 were of mild severity, 7 were of moderate severity, and no severe AE was reported. The large majority of events were dermatological in nature (see below). The two related non-dermatological events were both in the CD0271 0.3%/CD1579 2.5% Gel group. One was a case of eyelid erythema (coded as Eye Disorder) and the other was a case of paresthesia (coded as Nervous System Disorder).

Of the 17 AEs considered related to study medication, 15 were reported from 11 subjects in subgroup of subjects with moderate acne (baseline IGA=3), with 10 subjects in CD0271 0.3%/CD1579 2.5% Gel group, and one in Epiduo Gel group. Only 2 were reported from 2 subjects in subgroup of subjects with severe acne (baseline IGA=4). Both subjects are in the CD0271 0.3%/CD1579 2.5% Gel group.

In the CD0271 0.3%/CD1579 2.5% Gel group, the most common related AEs were Skin irritation [6 (2.8%) of subjects] and Skin burning sensation [2 (0.9%) of subjects]. These were all mild or moderate and did not lead to study discontinuation. One related AEs (atopic dermatitis flare) in the CD0271 0.3%/CD1579 2.5% Gel group led to subject discontinuation from the study. The onset of most of the related AEs in this group was in the first month of the study with only one occurring in the second month (facial irritation) and one in the third month (irritation anterior neck).

**Table 7 - Summary of Related Adverse Events**

| **System Organ Class/Preferred Term** | **CD0271 0.3%/ CD1579 2.5% Gel (N=217)** | **Epiduo Gel (N=217)** | **Vehicle Gel (N=69)** | **Total (N=503)** |
|---|---|---|---|---|
| | | | | |
| Total Number of AE(s) | 15 | 2 | 0 | 17 |
| | | | | |
| Total Number (%) of Subjects with AE(s) | 12 (5.5%) | 1 (0.5%) | 0 | 13 (2.6%) |
| | | | | |
| Skin and subcutaneous tissue disorders | 11 (5.1%) | 1 (0.5%) | 0 | 12 (2.4%) |
| Skin irritation | 6 (2.8%) | 0 | 0 | 6 (1.2%) |
| Pruritus | 1 (0.5%) | 1 (0.5%) | 0 | 2 (0.4%) |
| Skin burning sensation | 2 (0.9%) | 0 | 0 | 2 (0.4%) |
| Dermatitis atopic | 1 (0.5%) | 0 | 0 | 1 (0.2%) |
| Eczema | 1 (0.5%) | 0 | 0 | 1 (0.2%) |
| Erythema | 0 | 1 (0.5%) | 0 | 1 (0.2%) |
| Rash | 1 (0.5%) | 0 | 0 | 1 (0.2%) |
| Skin hypopigmentation | 1 (0.5%) | 0 | 0 | 1 (0.2%) |
| | | | | |
| Eye disorders | 1 (0.5%) | 0 | 0 | 1 (0.2%) |
| Erythema of eyelid | 1 (0.5%) | 0 | 0 | 1 (0.2%) |
| | | | | |
| Nervous system disorders | 1 (0.5%) | 0 | 0 | 1 (0.2%) |
| Paraesthesia | 1 (0.5%) | 0 | 0 | 1 (0.2%) |

### Serious Adverse events:

No deaths were reported for this study.

No SAEs were reported in the CD0271 0.3%/CD1579 2.5% Gel and in the vehicle group. Only one subject (in the Epiduo group) was reported to have one non-related moderate serious adverse event (generalized anxiety disorder) during the study.

### Adverse events leading to discontinuation:

There were two AEs leading to discontinuation during the study: one related adverse event (atopic dermatitis flare) was reported in one subject (0.5%) in the CD0271 0.3%/CD1579 2.5% Gel, one non-related adverse event (Worsening of acne) in one subject in the Epiduo group and no AEs leading to discontinuation in the vehicle arm.

### Severe adverse events:

No severe adverse events were reported in the CD0271 0.3%/CD1579 2.5% Gel and Epiduo group. A total of one non-related severe adverse event (Significantly lowered potassium level) was reported in vehicle arm in one (1.4%) subject.

### Adverse events of Special Interest:

In this study, The Adverse Event of Special Interest (AESI) was defined as suspected sensitization. A total of one AESI was reported in CD0271 0.3%/CD1579 2.5% Gel group in one subject (0.5%).The AESI (irritant dermatitis with a suspicion of sensitization) was considered related and mild in severity. It occurred on Day19 and lasted for 12 days. The subject was offered to be patch tested but refused and discontinued from the study.

### Local Tolerability:

Local tolerability parameters (erythema, scaling, dryness, stinging/burning) were evaluated on a 4-point scale with 0=none, 1=mild, 2=moderate, 3=severe at each visit. Table 8 and Table 9 provide the summary for the highest score worse than baseline for the overall Safety Population and for the Baseline IGA=4 subgroup.

For erythema, scaling and dryness, the incidences were higher in the CD0271 0.3%/CD1579 2.5% Gel group than in Epiduo Gel group, compared to the Vehicle Gel group. For stinging/burning, the incidences were comparable in CD0271 0.3%/CD1579 2.5% Gel group and in Epiduo Gel group, and were higher compared to the Vehicle Gel group.

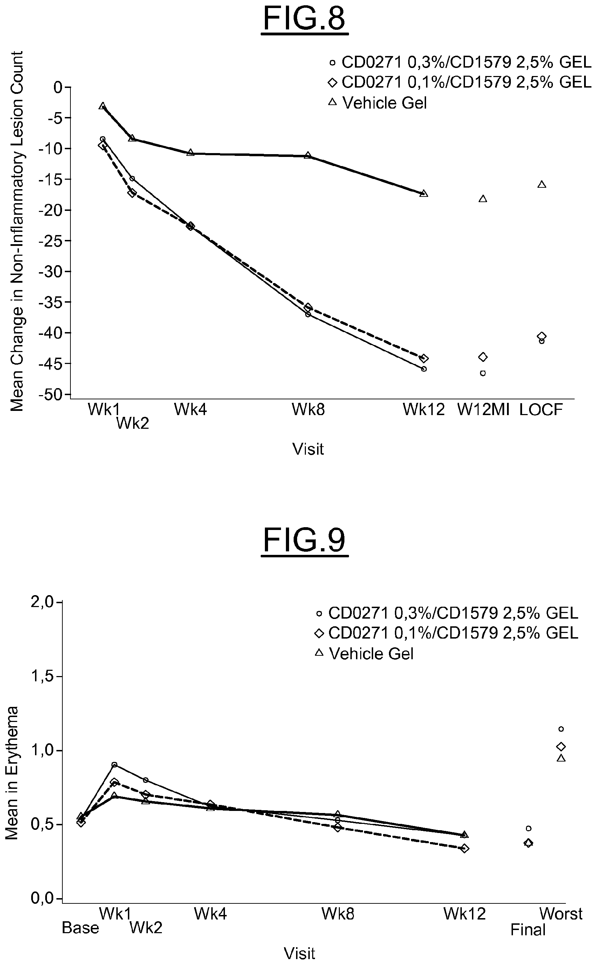

The mean scores of local tolerability signs and symptoms increased to a peak at week 1, thereafter decreased over time as shown in Figures 9 to 12. The peak scores were marginally higher with CD0271 0.3%/CD1579 2.5% Gel compared to Epiduo Gel. Signs and symptoms of local tolerability were mostly mild or moderate, with very few being severe.

**Table 8 - Highest Local Tolerability Score Worse Than Baseline (Safety Population)**

| **Highest local tolerability score Worse Than Baseline** | **CD0271 0.3%/CD 1579 2.5% Gel** | **Epiduo Gel** | **Vehicle Gel** |
|---|---|---|---|
| **Erythema** | **104/213 (48.8%)** | **93/212 (43.9%)** | **25/ 68 (36.8%)** |
| 1 = Mild | 59/213 (27.7%) | 58/212 (27.4%) | 20/ 68 (29.4%) |
| 2 = Moderate | 43/213 (20.2%) | 32/212 (15.1%) | 4/ 68 (5.9%) |
| 3 = Severe | 2/213 (0.9%) | 3/212 (1.4%) | 1/ 68 (1.5%) |
| **Scaling** | **116/213 (54.5%)** | **101/212 (47.6%)** | **21/ 68 (30.9%)** |
| 1 = Mild | 78/213 (36.6%) | 75/212 (35.4%) | 17/ 68 (25.0%) |
| 2 = Moderate | 36/213 (16.9%) | 25/212 (11.8%) | 4/ 68 (5.9%) |
| 3 = Severe | 2/213 (0.9%) | 1/212 (0.5%) | 0/ 68 (0.0%) |
| **Dryness** | **137/213 (64.3%)** | **132/212 (62.3%)** | **27/ 68 (39.7%)** |
| 1 = Mild | 100/213 (46.9%) | 102/212 (48.1%) | 23/ 68 (33.8%) |
| 2 = Moderate | 32/213 (15.0%) | 27/212 (12.7%) | 3/ 68 (4.4%) |
| 3 = Severe | 5/213 (2.3%) | 3/212 (1.4%) | 1/ 68 (1.5%) |
| **Stinging/burning** | **141/213 (66.2%)** | **138/212 (65.1%)** | **19/ 68 (27.9%)** |
| 1 = Mild | 88/213 (41.3%) | 88/212 (41.5%) | 16/ 68 (23.5%) |
| 2 = Moderate | 40/213 (18.8%) | 30/212 (14.2%) | 2/ 68 (2.9%) |
| 3 = Severe | 13/213 (6.1%) | 20/212 (9.4%) | 1/ 68 (1.5%) |

Local tolerability profiles are similar in the subgroup of subjects with severe acne (baseline IGA= 4).

**Table 9 - Highest Local Tolerability Score Worse Than Baseline in Subjects with Severe Acne (Baseline IGA=4, Safety Population)**

| **Highest local tolerability score Worse Than Baseline** | **CD0271 0.3%/CD 1579 2.5% Gel** | **Epiduo Gel** | **Vehicle Gel** |
|---|---|---|---|
| **Erythema** | **49/103 (47.6%)** | **50/111 (45.0%)** | **14/ 33 (42.4%)** |
| 1 = Mild | 27/103 (26.2%) | 26/111 (23.4%) | 11/ 33 (33.3%) |
| 2 = Moderate | 20/103 (19.4%) | 21/111 (18.9%) | 2/33 (6.1%) |
| 3 = Severe | 2/103 (1.9%) | 3/111 (2.7%) | 1/ 33 (3.0%) |
| **Scaling** | **57/103 (55.3%)** | **54/111 (48.6%)** | **8/ 33 (24.2%)** |
| 1 = Mild | 34/103 (33.0%) | 38/111 (34.2%) | 5/33 (15.2%) |
| 2 = Moderate | 22/103 (21.4%) | 15/111 (13.5%) | 3/ 33 (9.1%) |
| 3 = Severe | 1/103 (1.0%) | 1/111 (0.9%) | 0/ 33 (0.0%) |
| **Dryness** | **68/103 (66.0%)** | **66/111 (59.5%)** | **12/ 33 (36.4%)** |
| 1 = Mild | 48/103 (46.6%) | 51/111 (45.9%) | 9/ 33 (27.3%) |
| 2 = Moderate | 17/103 (16.5%) | 13/111 (11.7%) | 2/33 (6.1%) |
| 3 = Severe | 3/103 (2.9%) | 2/111 (1.8%) | 1/ 33 (3.0%) |
| **Stinging/burning** | **69/103 (67.0%)** | **73/111 (65.8%)** | **13/ 33 (39.4%)** |
| 1 = Mild | 41/103 (39.8%) | 49/111 (44.1%) | 11/ 33 (33.3%) |
| 2 = Moderate | 23/103 (22.3%) | 15/111 (13.5%) | 1/ 33 (3.0%) |
| 3 = Severe | 5/103 (4.9%) | 9/11 (8.1%) | 1/ 33 (3.0%) |

### DISCUSSION:

IGA success rate, a dichotomized parameter, in the severe population requires at least a three point change to Almost Clear and Clear. Here the difference between CD0271 0.3%/CD1579 2.5% Gel and Epiduo is the most prominent, perhaps reflecting on the more potent effect of the higher concentration of adapalene in the new product. Specifically, the treatment effect difference vs. vehicle in this outcome for CD0271 0.3%/CD1579 2.5% Gel is 20.1% and statistically significant (p=0.029), whereas the treatment effect difference vs. vehicle in this outcome for Epiduo gel is 8.8% and was not statistically significant (nominal p=0.443; post-hoc analysis not done as part of defined statistical plan)

It is interesting to note that skin related adverse events observed with CD0271 0.3%/CD1579 2.5% Gel were less frequent in the severe group, and this is the group that demonstrates the larger deltas in terms of treatment effect. This favorable benefit risk profiles argues well in favor of the use of this product in the severe population.

### CONCLUSION:

This study demonstrated that CD0271 0.3%/CD1579 2.5% Gel was superior to vehicle in the co-primary endpoints IGA success rate (clear/almost clear and two grade change) and change in Inflammatory and Non-inflammatory lesions counts. The statistical significance was consistently high (p<0.001) and the outcome clinically compelling. The study is robust, presenting consistent results in the PP population and sensitivity analyses.

In the subgroup of subjects with severe acne (IGA=4), CD0271 0.3%/CD1579 2.5% Gel was superior to Vehicle in IGA success rate (p=0.029), change in Inflammatory (p<0.001) and Non-inflammatory lesion counts (p<0.001). The outcome was supported by robust sensitivity analyses.

A trend of numerical superiority of CD0271 0.3%/CD1579 2.5% Gel compared to Epiduo Gel in IGA success rate, change in inflammatory and non-inflammatory lesion counts was observed in subjects with severe acne (IGA=4).

CD0271 0.3%/CD1579 2.5% Gel was well tolerated with an acceptable safety profile.

## Claims

1. A pharmaceutical composition comprising 0.3% by weight of adapalene or a pharmaceutically acceptable salt thereof and 2.5% by weight of benzoyl peroxide, as active ingredients, wherein the percents by weight are relative to the total weight of the composition, for its use by topical administration in the treatment of inflammatory acne lesions in a subject afflicted with having an Investigate Global Assessment of 4, wherein the composition is an aqueous gel.

2. The composition for its use according to claim 1, wherein said composition is administered once or twice daily for a period of 8 to 12 weeks.

3. The composition for its use according to claim 1, wherein said composition is administered once or twice daily for a period of 4 to 12 weeks.

4. The composition for its use according to anyone of claims 1 to 3, wherein the composition is an aqueous gel comprising a pH independent gelling agent.

5. The composition for use according to claim 4, wherein the pH independent gelling agent is a mixture of acrylamide/sodium acryloyldimethyl taurate copolymer and isohexadecane and polysorbate 80 (SIMULGEL 600) or a mixture of polyacrylamide and isoparaffin and Laureth-7 (SEPIGEL 305).

6. The composition for use according to claim 5, wherein it further comprises propylene glycol, glycerol and a poloxamer.

## Patentansprüche

1. Pharmazeutische Zusammensetzung, die 0,3 Gew.% Adapalen oder ein pharmazeutisch annehmbares Salz davon und 2,5 Gew.% Benzoylperoxid als Wirkstoffe umfasst, wobei sich die Gewichtsprozente auf das Gesamtgewicht der Zusammensetzung beziehen, zur Verwendung durch topische Verabreichung bei der Behandlung von entzündlichen Akne-Läsionen bei einem Subjekt, das von Akne betroffen ist und einen Ivestigator Global Assessment von 4 aufweist, wobei die Zusammensetzung ein wässriges Gel ist.

2. Zusammensetzung zur Verwendung nach Anspruch 1, wobei die Zusammensetzung für einen Zeitraum von 8 bis 12 Wochen einmal oder zweimal täglich verabreicht wird.

3. Zusammensetzung zur Verwendung nach Anspruch 1, wobei die Zusammensetzung für einen Zeitraum von 4 bis 12 Wochen einmal oder zweimal täglich verabreicht wird.

4. Zusammensetzung zur Verwendung nach einem der Ansprüche 1 bis 3, wobei die Zusammensetzung ein wässriges Gel ist, das ein pH-unabhängiges Geliermittel umfasst.

5. Zusammensetzung zur Verwendung nach Anspruch 4, wobei das pH-unabhängige Geliermittel eine Mischung aus Acrylamid/Natriumacryloyldimethyltaurat-Copolymer und Isohexadecan und Polysorbat 80 (SIMULGEL 600) oder eine Mischung aus Polyacrylamid und Isoparaffin und Laureth-7 (SEPIGEL 305) ist.

6. Zusammensetzung 1 zur Verwendung nach Anspruch 5, wobei diese ferner Propylenglykol, Glycerin und ein Poloxamer umfasst.

## Revendications

1. Composition pharmaceutique comprenant 0,3 % en poids d'adapalène ou d'un sel pharmaceutiquement acceptable de celui-ci et 2,5 % en poids de peroxyde de benzoyle, en tant que substances actives, dans laquelle les pourcentages en poids sont relatifs au poids total de la composition, pour son utilisation par administration topique dans le traitement de lésions acnéiques inflammatoires chez un sujet atteint d'acné ayant une évaluation globale de chercheur de 4, dans laquelle la composition est un gel aqueux.

2. Composition pour son utilisation selon la revendication 1, dans laquelle ladite composition est appliquée une ou deux fois par jour pendant une période de 8 à 12 semaines.

3. Composition pour son utilisation selon la revendication 1, dans laquelle ladite composition est appliquée une ou deux fois par jour pendant une période de 4 à 12 semaines.

4. Composition pour son utilisation selon l'une quelconque des revendications 1 à 3, dans laquelle la composition est un gel aqueux comprenant un agent gélifiant indépendant du pH.

5. Composition à utiliser selon la revendication 4, dans laquelle l'agent gélifiant indépendant du pH est un mélange de copolymère acrylamide/acryloyldiméthyl taurate de sodium et d'isohexadécane et de polysorbate 80 (SIMULGEL 600) ou un mélange de polyacrylamide et d'isoparaffine et de Laureth-7 (SEPIGEL 305).

6. Composition à utiliser selon la revendication 5, dans laquelle elle comprend en outre du propylène glycol, du glycérol et un poloxamère.
